Europäisches Patentamt

European Patent Office

Office européen des brevets

(19)

(11) Numéro de publication: **0 091 528**
**B1**

# (12) FASCICULE DE BREVET EUROPÉEN

(45) Date de publication du fascicule du brevet:
23.07.86

(21) Numéro de dépôt: **82400649.8**

(22) Date de dépôt: **06.04.82**

(51) Int. Cl.⁴: **C 07 D 207/09**, A 61 K 31/40

(54) Nouvel agent utilisable pour le traitement de certaines formes de la dépression constitué par le N-(1-propyl-2-pyrrolidylméthyl)-2-méthoxy 5-sulfamoyl benzamide.

(43) Date de publication de la demande:
**19.10.83 Bulletin 83/42**

(45) Mention de la délivrance du brevet:
**23.07.86 Bulletin 86/30**

(84) Etats contractants désignés:
**AT BE CH FR GB LI LU NL SE**

(56) Documents cité:
**FR-A-2 252 336**
**FR-A-2 314 178**
**FR-M-5 916**

(73) Titulaire: **SOCIETE D'ETUDES SCIENTIFIQUES ET INDUSTRIELLES DE L'ILE DE FRANCE, 46, Boulevard de Latour- Maubourg, F-75340 Paris Cedex 07 (FR)**

(72) Inventeur: **Thominet, Michel, 82, rue Bonaparte, F-75006 Paris (FR)**
Inventeur: **Laville, Claude, 4, rue Jean- Marie Jego, F-75013 Paris (FR)**

(74) Mandataire: **Gallochat, Alain, SOCIETE D'ETUDES SCIENTIFIQUES ET INDUSTRIELLES DE L'ILE DE FRANCE 46, Boulevard de Latour Maubourg, F-75340 Paris Cedex 07 (FR)**

EP 0 091 528 B1

## Description

La présente invention concerne un nouvel agent utilisable dans le traitement de certaines formes de la dépression constitué par le N-(1-propyl-2-pyrrolidylméthyl)-2-méthoxy-5-sulfamoyl benzamide.

A titre indicatif, ce composé peut être synthétisé de la façon suivante:

On mélange 40 g d'acide sulfurique concentré et 800 g d'alcool absolu (99°5 à 99°9), et on ajoute 231 g d'acide 2-méthoxy-5-sulfamoyl benzoïque connu par ailleurs; on chauffe à reflux la suspension pendant 4 à 5 heures. La dissolution complète se produit au bout de 1 h.30 à 2 heures.

On distille alors la majeure partie de l'alcool au bain-marie, jusqu'à début de cristallisation.

On dilue encore chaud avec 2400 ml d'eau froide ce qui achève la cristallisation de l'ester.

On laisse refroidir, neutralise jusqu'à pH 8 environ avec du carbonate de soude en poudre (25-30 g).

On essore, lave, sèche à 50-55°.

. Poids obtenu = 245 g

. Rendement = 95 %

. Point de fusion au bloc = 150-151°

Dans un ballon d'un litre muni d'un agitateur et d'un thermomètre on introduit 65 g de l'ester précédemment obtenu et 300 ml de glycol éthylènique; on chauffe vers 90° jusqu'à complète dissolution et on refroidit à50°C. L'ester recristallise; on ajoute alors 45 g de 1-propyl-2-aminométhyl pyrrolidine connue par ailleurs (par exemple décrite dans le brevet allemand n° 1966 195 au nom de la demanderesse), et on maintient la suspension obtenue à 50-55° pendant 92 heures (au bout de 23 heures tout est dissous et en fin de réaction on observe un début de cristallisation).

La réaction terminée, on ajoute 500 ml d'eau pour terminer la cristallisation du produit. Il est essoré au bout de 4 heures, lavé à l'eau et séché à 50°

. Poids obtenu = 69 g

. Rendement = 77 %

. F = 167-171°

Le produit obtenu beige foncé est redissous dans l'eau et l'acide acétique nécessaire à la neutralisation. La solution trouble est filtrée avec du noir et la base est reprécipitée par addition de $NH_4OH$. La base d'abord liquide se solidifie. Elle est essorée, lavée à l'eau et séchée à50°.

. Poids obtenu = 66 g

. Rendement purification = 95 %

. Rendement total = 73

La base est mise en suspension dans 198 ml d'eau et 18,5 ml de HCl concentré sont ajoutés à chaud jusqu'à virage du rouge de méthyle etjusqu'à complète dissolution à chaud.

Par refroidissement le chlorhydrate cristallise. Il est essoré, lavé avec 10 ml d'eau glacée et séché à 50°.

. Poids obtenu = 68 g

. Rendement = 94 %

. P.M. ($NO_3Ag\ N_{10}$) = 393

. F = 240-245°

Dans un grignard on introduit 40 litres d'eau déminéralisée et en agitant on ajoute 10 kg de chlorhydrate de N-(1-propyl-2-pyrrolidinylméthyl)-2-méthoxy-5-sulfamoyl benzamide.

On chauffe jusqu'à dissolution totale (80-90°) et ajoute 4 litres d'ammoniaque à 34 %. La base précipite. On continue d'agiter et refroidit par courant d'eau. Vers 25°, on coule dans deux bassines en acier inoxydable que l'on laisse pendant la nuit à la chambre froide à + 4°.

Le lendemain on essore sur essoreuse, lave abondamment à l'eau (on vérifie l'absence des ions C1⁻ dans l'eau de lavage) et on sèche à 50°.

Le produit est alors tamisé.

. Rendement = 8,800 kg (97 %)

. PF = 174°

. Produit conforme aux normes.

Le composé selon l'invention fait partie de la famille des benzamides et son homologue le plus proche est le N-(1-éthyl-2-pyrrolidinyl-méthyl)-2-méthoxy-5-sulfamoyl benzamide, dont la Dénomination Commune Internationale est le Sulpiride.

D'une façon générale, ces dérivés benzamides interfèrent dans les mécanismes dopaminergiques du système nerveux. Leurs effets bloquantssur les récepteurs dopamine centraux leur confèrent des propriétés neuroleptiques. Leur effet bloquant sur les récepteurs dopamine du centre du vomissement se traduit par un puissant antagonisme à l'égard de l'effet émétisant decertains agonistes dopaminergiques, tels que l'apomorphine et les alcaloïdes de l'ergot de seigle.

La toxicité et les propriétés pharmacologiques du composé selon la présente invention et du Sulpiride, antérieurement décrit, ont été comparées faisant apparaître les résultats suivants:

Au plan toxicologique, la détermination des DL 50 des deux produits administrés chez la souris par différentes voies ne révèle aucune différence majeure, le Sulpiride étant toutefois un peu moins toxique par voie buccale.

**0 091 528**

| DL 50 mg/kg | Composé selon l'invention | Sulpiride |
|---|---|---|
| I.V. | 51,7—52,6 | 43,5—44 |
| S.C. | 337—363 | 372 |
| I.P. | 181—203 | 210 |
| P.O. | 1411—1423 | 1800—2891 |

Au plan pharmacologique, les interférences des deux composés dans le système dopamine ont été comparées par différents tests adaptés à la mise en évidence des effets antagonistes et agonistes. Il en résulte que:

1) - le composé selon l'invention et le Sulpiride sont de très puissants antagonistes dans le test de l'emesis chez le chien où les vomissements sont provoqués par l'agoniste apomorphine.

Les valeurs des DE 50 montrent que les activités des deux composés sont équivalentes.

| Pouvoir antiémétique chez le chien | Composé selon l'invention | Sulpiride |
|---|---|---|
| DE 50 S.C. $\gamma$/kg | 4,1 $\gamma$/kg | 3,5—4,8 $\gamma$/kg |

2) - Les deux composés ne se distinguent pas non plus quand leur propriété antagoniste dopaminergique centrale est jugée par exemple d'après l'inhibition qu'ils provoquent du mouvement de redressement induit chez la souris par l'apomorphine (1 mg/kg S.C.).

Les doses inhibitrices 50 de ce mouvement sont voisines pour les deux composés, respectivement

Sulpiride 22 mg/kg I.P.

Composé selon l'invention 32 mg/kg I.P.

3) - Par contre, les deux composés se distinguent considérablement l'un de l'autre dans des tests visant, à l'inverse, à déceler une propriété agoniste dopaminergique.

A cet effet, le composé de l'invention a été étudié vis-à-vis de la potentialisation des stéréotypies induites chez le Rat par deux agonistes dopaminergiques, l'apomorphine (agoniste direct) et la d-amphétamine (agoniste indirect).

Dans les deux essais, la dose d'agoniste est choisie telle qu'elle n'entraîne pas, chez tous les animaux d'expérience, l'apparition de mouvements stéréotypés de la sphère buccale de type léchage ou mâchonnement (Apomorphine: 0,250 mg.kg$^{-1}$ S.C. - d-amphétamine: 3 mg.kg$^{-1}$ I.P.).

L'observation de ce comportement stéréotypé chez un plus grand nombre d'animaux consecutif à l'administration de doses croissantes du composé de l'invention, injectées par voie I.P. 30 minutes avant l'agoniste, traduit un renforcement dopaminergique.

Les $DE_{50}$ déterminées par la methode de Bliss assortie des limites de confiance à 95% sont de 10,8 et 8,9 mg.kg. $^{-1}$ I.P. vis-à-vis de l'apomorphine et 1,4 et 2,6 mg.kg.$^{-1}$ vis-à-vis de la d-amphétamine.

Dans les mêmes conditions expérimentales, le SULPIRIDE est inactif.

Les tableaux suivants (I et II) rassemblent les résultats du compose de l'invention, comparativement à ceux du SULPIRIDE.

3

**Tableau I** : Action vis-à-vis de l'Apomorphine 0,250 mg.kg⁻¹ S.C.

| PRODUITS | Doses mg.kg⁻¹ I.P. 30 min.avant agoniste | | Nombre d'animaux positifs/16 | | % d'activité | | DE$_{50}$ mg.kg⁻¹ I.P. base | |
|---|---|---|---|---|---|---|---|---|
| N° Essai | I | II | I | II | I | II | I | II |
| Composé selon l'invention | 0 | 0 | 3 | 1 | | | 8,95 [8,00−10,45] | 10,75 [9,03−14,11] |
| | 4 | 4 | 6 | 2 | 23 | 7 | | |
| | 8 | 8 | 9 | 6 | 46 | 33 | | |
| | 16 | 16 | 12 | 11 | 69 | 67 | | |
| SULPIRIDE | 0 | | 3 | | | | | |
| | 4 | | 5 | | 15 | | inactif | |
| | 8 | | 7 | | 31 | | | |
| | 16 | | 5 | | 15 | | | |

**Tableau II** : Action vis-à-vis de la d-Amphétamine 3 mg.kg⁻¹ I.P.

| PRODUITS | Doses mg.kg⁻¹ I.P. 30 min.avant agoniste | | Nombre d'animaux positifs/16 | | % d'activité | | DE$_{50}$ mg.kg⁻¹ I.P. base | |
|---|---|---|---|---|---|---|---|---|
| N° Essai | I | II | I | II | I | II | I | II |
| Composé selon l'invention | 0 | 0 | 0 | 0 | | | 2,60 [2,47−2,84] | 1,44 [1,25−2,61] |
| | 2 | 1 | 6 | 6 | 38 | 38 | | |
| | 4 | 4 | 11 | 13 | 69 | 81 | | |
| | 8 | 16 | 14 | 15 | 88 | 94 | | |
| SULPIRIDE | 0 | | 0 | | | | | |
| | 2 | | 1 | | 6 | | inactif | |
| | 4 | | 2 | | 13 | | | |
| | 8 | | 1 | | 6 | | | |

Cette propriété du composé selon l'invention de révéler ou de potentialiser ces effets particuliers des agonistes dopaminergiques permet de le rapprocher de certains composés (par exemple tricycliques) connus pour leurs propriétés antidépressives en clinique humaine et présentant aussi ces propriétés agonistes dopaminergiques.

En conséquence, un essai clinique comparatif en double aveugle a été mené mettant en oeuvre le composé selon l'invention et l'IMIPRAMINE, produit de référence indiscutable dans le traitement de certains états dépressifs.

Les posologies retenues ont été fixées:

. pour l'IMIPRAMINE en fonction des données acquises et de la posologie préconisée par le fabricant, c'est-à-dire: 150 mg par jour, dose atteinte progressivement en 5 jours;

. pour le composé selon l'invention, à partir des données recueillies par les études antérieures, c'est-à-dire: 450 mg par jour.Bien que cette posologie puisse, du fait de l'excellente tolérance du produit, être administrée d'emblée, pour se plier aux règles du double-aveugle et respecter le mode d'administration optimum pour l'IMIPRAMINE, cette dose n'a également été atteinte que le 5ème jour après deux paliers successifs de: 150 et 300 mg par jour. Toutefois d'autres études permettent de penser que des doses plus faibles du composé selon l'invention aboutissent à des résultats similaires.

Ces doses ont été administrées pendant 28 jours consécutifs en monothérapie, dans toute la mesure du possible.

Pouvaient entrer dans l'étude, les sujets présentant une dépression authentique c'est-à-dire atteignant le score de 36 à la version simplifiée à 17 items de l'échelle de Hamilton.

La comparaison entre les deux produits a porté sur l'efficacité thérapeutique et sur la tolérance, 34 cas ayant été analysés. Pour établir cette comparaison, le protocole prévoyait trois bilans:

. avant le début du traitement (J0)

. au 10ème jour (J10)

. au 28ème jour (J28)

Chacun de ces bilans comportait obligatoirement:

. une B.P.R.S.

. une échelle de Hamilton (26 items)

. une appréciation par l'expert de l'"intensité du syndrome dépressif"

. une recherche des troubles somatiques associés à l'état dépressif.

De plus, à J0 et à J28, il a été pratiqué une auto-appréciation de son état par le malade (S.D.S.) et un bilan biologique standard.

Enfin, en fin de traitement, il a été convenu d'exprimer une impression subjective globale sur l'intérêt du traitement pour chaque observation.

L'analyse statistique des données ainsi recueillies permet d'aboutir aux conclusions suivantes:

* les deux produits améliorent de façon significative les scores des malades quelle que soit la façon d'appréhender l'état clinique. On peut donc affirmer que tous deux peuvent être utilisés dans le traitement de certaines formes de la dépression; en particulier le composé selon l'invention se révèle particulièrement efficace sur la thymie, l'inhibition psychomotrice et la prise de conscience de l'état morbide.

* l'efficacité du composé selon l'invention est au moins égale à celle de l'IMIPRAMINE dans lesdites formes, puisque l'amélioration obtenue avec ledit composé est toujours plus ou moins supérieure à celle obtenue sous IMIPRAMINE;

* le composé selon l'invention agit plus rapidement sur l'état dépressif que l'IMIPRAMINE, cette supériorité étant toujours plus nette au 10ème jour qu'au 28ème ainsi que le montrent les seuils de significativité obtenus avec:

- la B.P.R.S.:

.. $p < 0,02$ au lieu de $p < 0,03$

- l'échelle d'Hamilton:

.. $p < 0,06$ au lieu de $p < 0,09$

- l'appréciation de l'intensité du syndrome dépressif:

.. $p < 0,01$ au lieu de $p < 0,09$

C'est un point qu'il est essentiel de souligner; en effet il est classiquement reproché aux imipraminiques d'agir avec une certaine latence d'une douzaine de jours en moyenne.

* la tolérance tant clinique que biologique des deux produits, a montré là encore, une supériorité du composé selon l'invention: moindre fréquence de l'apparition ou de l'aggravation d'une symptomatologie somatique, émergeant sous traitement, en particulier: troubles de la série anticholinergique nettement plus importants et plus sévères dans le groupe IMIPRAMINE (nécessitant pour ce produit une surveillance particulière en cas d'hypertrophie prostatique, d'hypotension artérielle et chez les malades présentant des antécédents cardiovasculaires athéromateux).

Globalement, on peut donc affirmer, au terme de cette étude comparative que le composé selon l'invention est réellement digne de figurer parmi les thérapeutiques de la dépression, puisque doué d'une activité:

. au moins égale à celle de l'IMIPRAMINE

. apparaissant plus rapidement

. provoquant moins d'effets somatiques indésirables.

Il est bien entendu que dans tout ce qui précède le composé selon l'invention doit être considéré soit sous forme de base ou bien de ses sels d'ammonium quaternaire, son N-oxyde, ses isomères, ainsi que leurs sels d'addition d'acide pharmacologiquement acceptables.

**Revendications** pour les Etats contractants: BE, CH, FR, GB, LI, LU, NL, SE

1 - N-(1-propyl-2-pyrrolidylméthyl)-2-méthoxy-5-sulfamoyl benzamide, ses sels d'ammonium quaternaire, son N-oxyde, ses isomères, ainsi que leurs sels d'addition d'acide pharmacologiquement acceptables.

2 - Composition pharmaceutique caractérisée en ce qu'elle comprend à titre de principe actif le composé selon la revendication 1 avec un support pharmacologiquement acceptable.

3 - A titre de médicament nouveau, notamment utilisable dans le traitement de la dépression, le composé selon la revendication 1.

**Revendication** pour l'Etat contractant AT

1. Procédé de préparation du N-(1-propyl 2 pyrrolidinylméthyl) 2-méthoxy 5-sulfamoyl benzamide d'addition d'acides pharmacologiquement acceptables, de ses sels d'ammonium quaternaire, de son N-oxyde et de ses isomères, qui consiste à traiter le 2-methoxy 5-sulfamoyl benzoate d'éthyle par la 1-propyl 2-amino-méthyl pyrrolidine.

**0 091 528**

**Patentansprüche** für die Vertragsstaaten: BE, CH, GB, LI, LU, NL, SE

1. N-(1-Fropyl-2-pyrrolidylmethyl)-2-methoxy-5-sulfamoyl-benzamid, seine quartären Ammoniumsalze, sein N-Oxid, seine Isomeren sowie pharmakologisch verträgliche Additionssalze derselben mit Säuren.
2. Pharmazeutische Zusammensetzung, dadurch gekennzeichnet, daß sie als Wirkstoff eine Verbindung nach Anspruch zusammen mit einem pharmakologisch verträglichen Träger enthält.
3. Medikament, insbesondere zur Behandlung von Depressionen, gekennzeichnet durch eine Verbindung nach Anspruch 1.

**Patentanspruch** für den Vertragsstaat: AT

1. Verfahren zur Herstellung von N-(1-Propyl-2-pyrrolidylmethyl)-2-methoxy-5-sulfamoylbenzamid, seiner quartären Ammoniumsalze, seines N-Oxids, seiner Isomeren sowie pharmakologisch verträglicher Additionssalze derselben mit Säuren, dadurch gekennzeichnet, daß man 2-Methoxy-5-sulfamoylbenzoesäure ethylester mit 1-Propyl-2-amino-methylpyrrolidin umsetzt.

**Claims** for the contracting states: BE, CH, FR, GB, LI, LU, NL, SE

1. N-(1-propyl-2-pyrrolidylmethyl)-2-methoxy-5-sulfamoyl benazamide, the quaternary ammonium salts thereof, the N-oxide thereof, the isomers thereof, and their pharmacologically acceptable acid addition salts.
2. A pharmaceutical composition characterised in that it comprises as active ingredient the compound according to claim 1 with a pharmacologically acceptable carrier.
3. As a novel medicament which can be used in particular in treating depression, the compound according to claim 1.

**Claim** for the Contracting state: AT

1. A process for the preparation of N-(1-propyl-2-pyrrolidylmethyl)-2-methoxy-5-sulfamoyl benzamide, the quaternary ammonium salts thereof, the N-oxide thereof, the isomers thereof and their pharmacologically acceptable acid addition salts, which comprises treating ethyl 2-methoxy-5-sulfamoyl benzoate with 1-propyl-2-aminomethyl pyrrolidine.